# EUROPEAN PATENT APPLICATION

(11) **EP 2 537 527 A2**
(43) Date of publication of application: **26.12.2012**
(21) Application number: 12184773.5
(22) Date of filing: 09.03.2006
(51) Int. Cl.: A61K 38/21

(54) **Uses of recombinant super-compound interferons**

(30) Priority: 09.03.2005 US 659925 P
(62) Divisional of application: 06795349.7
(71) Applicant: Wei, Guangwen, Chengdu, Sichuan 610017 (CN)
(72) Inventor: Wei, Guangwen, Chengdu, Sichuan 610017 (CN)
(74) Representative: Capasso, Olga

(57) **Abstract**

This invention provides different uses of recombinant super-compound interferons (rSIFN-cos) and its equivalent with changed spatial configuration, high efficacy and low side effects. Therefore, high dose of rSIFN-co may be used. One characteristic of rSIFN-co is its ability to inhibit the HBV DNA duplication and secretion of HBsAg and HBeAg in *in vitro* pharmacological studies. The cytotoxic effect of rSIFN-co is only one-eighth (1/8) of currently clinically available interferons but its anti-viral effect is approximately five to twenty (5-20) times higher, and when used in vivo it has a broader spectrum of clinical applications and longer biofeedback response. This invention further provides super-compound interferon or its equivalent, synthesis of artificial gene with codon preference which codes for said rSIFN-co and its equivalent, vector comprising said gene and appropriate expression system for expression of said rSIFN-co. Finally this invention provides the super-compound interferon (rSIFN-co) and its equivalent, a process to produce same and uses thereof.

SEQUENCE LISTING

<110>  Wei, Guangwen

<120>  USES OF RECOMBINANT SUPER-COMPOUND INTERFERONS

<130>  BE 118583

<150>  60/659,925
<151>  2005-03-09

<160>  8

<170>  PatentIn version 3.5

<210>  1
<211>  167
<212>  PRT
<213>  Artificial Sequence

<220>
<223>  Synthetic Construct

<400>  1

Met Cys Asp Leu Pro Gln Thr His Ser Leu Gly Asn Arg Arg Ala Leu
1               5                   10                  15

Ile Leu Leu Ala Gln Met Arg Arg Ile Ser Pro Phe Ser Cys Leu Lys
            20                  25                  30

Asp Arg His Asp Phe Gly Phe Pro Gln Glu Glu Phe Asp Gly Asn Gln
        35                  40                  45

Phe Gln Lys Ala Gln Ala Ile Ser Val Leu His Glu Met Ile Gln Gln
    50                  55                  60

Thr Phe Asn Leu Phe Ser Thr Lys Asp Ser Ser Ala Ala Trp Asp Glu
65                  70                  75                  80

Ser Leu Leu Glu Lys Phe Tyr Thr Glu Leu Tyr Gln Gln Leu Asn Asp
                85                  90                  95

Leu Glu Ala Cys Val Ile Gln Glu Val Gly Val Glu Glu Thr Pro Leu
            100                 105                 110

Met Asn Val Asp Ser Ile Leu Ala Val Lys Lys Tyr Phe Gln Arg Ile
        115                 120                 125

Thr Leu Tyr Leu Thr Glu Lys Lys Tyr Ser Pro Cys Ala Trp Glu Val
    130                 135                 140

Val Arg Ala Glu Ile Met Arg Ser Phe Ser Leu Ser Thr Asn Leu Gln
145                 150                 155                 160

Glu Arg Leu Arg Arg Lys Glu
                165

<210>  2
<211>  504
<212>  DNA
<213>  Artificial Sequence

<220>
<223>  Synthetic Construct

<220>
<221>  CDS
<222>  (1)..(501)

<400>  2
atg tgc gac ctg ccg cag acc cac tcc ctg ggt aac cgt cgt gct ctg        48
Met Cys Asp Leu Pro Gln Thr His Ser Leu Gly Asn Arg Arg Ala Leu
1               5                   10                  15

atc ctg ctg gct cag atg cgt cgt atc tcc ccg ttc tcc tgc ctg aaa        96
Ile Leu Leu Ala Gln Met Arg Arg Ile Ser Pro Phe Ser Cys Leu Lys
            20                  25                  30

gac cgt cac gac ttc ggt ttc ccg cag gaa gaa ttc gac ggt aac cag       144
Asp Arg His Asp Phe Gly Phe Pro Gln Glu Glu Phe Asp Gly Asn Gln
        35                  40                  45

ttc cag aaa gct cag gct atc tcc gtt ctg cac gaa atg atc cag cag       192
Phe Gln Lys Ala Gln Ala Ile Ser Val Leu His Glu Met Ile Gln Gln
    50                  55                  60

acc ttc aac ctg ttc tcc acc aaa gac tcc tcc gct gct tgg gac gaa       240
Thr Phe Asn Leu Phe Ser Thr Lys Asp Ser Ser Ala Ala Trp Asp Glu
65                  70                  75                  80

tcc ctg ctg gaa aaa ttc tac acc gaa ctg tac cag cag ctg aac gac       288
Ser Leu Leu Glu Lys Phe Tyr Thr Glu Leu Tyr Gln Gln Leu Asn Asp
                85                  90                  95

ctg gaa gct tgc gtt atc cag gaa gtt ggt gtt gaa gaa acc ccg ctg       336
Leu Glu Ala Cys Val Ile Gln Glu Val Gly Val Glu Glu Thr Pro Leu
            100                 105                 110

atg aac gtt gac tcc atc ctg gct gtt aaa aaa tac ttc cag cgt atc       384
Met Asn Val Asp Ser Ile Leu Ala Val Lys Lys Tyr Phe Gln Arg Ile
        115                 120                 125

acc ctg tac ctg acc gaa aaa aaa tac tcc ccg tgc gct tgg gaa gtt       432
Thr Leu Tyr Leu Thr Glu Lys Lys Tyr Ser Pro Cys Ala Trp Glu Val
    130                 135                 140

gtt cgt gct gaa atc atg cgt tcc ttc tcc ctg tcc acc aac ctg cag       480
Val Arg Ala Glu Ile Met Arg Ser Phe Ser Leu Ser Thr Asn Leu Gln
145                 150                 155                 160

gaa cgt ctg cgt cgt aaa gaa taa                                       504
Glu Arg Leu Arg Arg Lys Glu
                165

<210>  3

<211>  504
<212>  DNA
<213>  Artificial Sequence

<220>
<223>  Synthetic Construct

<400>  3
tacacgctgg acggcgtctg ggtgagggac ccattggcag cacgagacta ggacgaccga      60

gtctacgcag catagagggg caagaggacg gactttctgg cagtgctgaa gccaaagggc     120

gtccttctta agctgccatt ggtcaaggtc tttcgagtcc gatagaggca agacgtgctt     180

tactaggtcg tctggaagtt ggacaagagg tggtttctga ggaggcgacg aaccctgctt     240

agggacgacc tttttaagat gtggcttgac atggtcgtcg acttgctgga ccttcgaacg     300

caataggtcc ttcaaccaca acttctttgg ggcgactact tgcaactgag gtaggaccga     360

caatttttta tgaaggtcgc atagtgggac atggactggc ttttttttat gaggggcacg     420

cgaacccttc aacaagcacg actttagtac gcaaggaaga gggacaggtg gttggacgtc     480

cttgcagacg cagcatttct tatt                                            504


<210>  4
<211>  120
<212>  PRT
<213>  Artificial Sequence

<220>
<223>  Synthetic Construct

<400>  4

Met Cys Asp Leu Pro Gln Thr His Ser Leu Gly Asn Arg Arg Ala Leu
1               5                   10                  15


Ile Leu Leu Ala Gln Met Arg Arg Ile Ser Pro Phe Ser Cys Leu Lys
            20                  25                  30


Asp Arg His Asp Phe Gly Phe Pro Gln Glu Glu Phe Asp Gly Asn Gln
        35                  40                  45


Phe Gln Lys Ala Gln Ala Ile Ser Val Leu His Glu Met Ile Gln Gln
    50                  55                  60


Thr Phe Asn Leu Phe Ser Thr Lys Asp Ser Ser Ala Ala Trp Asp Glu
65                  70                  75                  80


Ser Leu Leu Glu Lys Phe Tyr Thr Glu Leu Tyr Gln Gln Leu Asn Asp
                85                  90                  95


Leu Glu Ala Cys Val Ile Gln Glu Val Gly Val Glu Glu Thr Pro Leu
            100                 105                 110

Met Asn Val Asp Ser Ile Leu Ala
        115                 120

<210>  5
<211>  360
<212>  DNA
<213>  Artificial Sequence

<220>
<223>  Synthetic Construct

<220>
<221>  CDS
<222>  (1)..(360)

<400>  5
atg tgt gat tta cct caa act cat tct ctt ggt aac cgt cgc gct ctg      48
Met Cys Asp Leu Pro Gln Thr His Ser Leu Gly Asn Arg Arg Ala Leu
1               5                   10                  15

att ctg ctg gca cag atg cgt cgt att tcc ccg ttt agc tgc ctg aaa      96
Ile Leu Leu Ala Gln Met Arg Arg Ile Ser Pro Phe Ser Cys Leu Lys
            20                  25                  30

gac cgt cac gac ttc ggc ttt ccg caa gaa gag ttc gat ggc aac caa      144
Asp Arg His Asp Phe Gly Phe Pro Gln Glu Glu Phe Asp Gly Asn Gln
        35                  40                  45

ttc cag aaa gct cag gca atc tct gta ctg cac gaa atg atc caa cag      192
Phe Gln Lys Ala Gln Ala Ile Ser Val Leu His Glu Met Ile Gln Gln
    50                  55                  60

acc ttc aac ctg ttt tcc act aaa gac agc tct gct gct tgg gac gaa      240
Thr Phe Asn Leu Phe Ser Thr Lys Asp Ser Ser Ala Ala Trp Asp Glu
65                  70                  75                  80

agc ttg ctg gag aag ttc tac act gaa ctg tat cag cag ctg aac gac      288
Ser Leu Leu Glu Lys Phe Tyr Thr Glu Leu Tyr Gln Gln Leu Asn Asp
                85                  90                  95

ctg gaa gca tgc gta atc cag gaa gtt ggt gta gaa gag act ccg ctg      336
Leu Glu Ala Cys Val Ile Gln Glu Val Gly Val Glu Glu Thr Pro Leu
            100                 105                 110

atg aac gtc gac tct att ctg gca                                      360
Met Asn Val Asp Ser Ile Leu Ala
        115                 120

<210>  6
<211>  360
<212>  DNA
<213>  Artificial Sequence

<220>
<223>  Synthetic Construct

<400>  6
tacacactaa atggagtttg agtaagagaa ccattggcag cgcgagacta agacgaccgt      60

gtctacgcag cataaagggg caaatcgacg gactttctgg cagtgctgaa gccgaaaggc      120

gttcttctca agctaccgtt ggttaaggtc tttcgagtcc gttagagaca tgacgtgctt 180

tactaggttg tctggaagtt ggacaaaagg tgatttctgt cgagacgacg aaccctgctt 240

tcgaacgacc tcttcaagat gtgacttgac atagtcgtcg acttgctgga ccttcgtacg 300

cattaggtcc ttcaaccaca tcttctctga ggcgactact tgcagctgag ataagaccgt 360

<210> 7
<211> 507
<212> DNA
<213> Artificial Sequence

<220>
<223> Synthetic Construct

<220>
<221> CDS
<222> (1)..(501)

<400> 7
atg tgt gat tta cct caa act cat tct ctt ggt aac cgt cgc gct ctg 48
Met Cys Asp Leu Pro Gln Thr His Ser Leu Gly Asn Arg Arg Ala Leu
1               5                   10                  15

att ctg ctg gca cag atg cgt cgt att tcc ccg ttt agc tgc ctg aaa 96
Ile Leu Leu Ala Gln Met Arg Arg Ile Ser Pro Phe Ser Cys Leu Lys
            20                  25                  30

gac cgt cac gac ttc ggc ttt ccg caa gaa gag ttc gat ggc aac caa 144
Asp Arg His Asp Phe Gly Phe Pro Gln Glu Glu Phe Asp Gly Asn Gln
        35                  40                  45

ttc cag aaa gct cag gca atc tct gta ctg cac gaa atg atc caa cag 192
Phe Gln Lys Ala Gln Ala Ile Ser Val Leu His Glu Met Ile Gln Gln
    50                  55                  60

acc ttc aac ctg ttt tcc act aaa gac agc tct gct gct tgg gac gaa 240
Thr Phe Asn Leu Phe Ser Thr Lys Asp Ser Ser Ala Ala Trp Asp Glu
65                  70                  75                  80

agc ttg ctg gag aag ttc tac act gaa ctg tat cag cag ctg aac gac 288
Ser Leu Leu Glu Lys Phe Tyr Thr Glu Leu Tyr Gln Gln Leu Asn Asp
                85                  90                  95

ctg gaa gca tgc gta atc cag gaa gtt ggt gta gaa gag act ccg ctg 336
Leu Glu Ala Cys Val Ile Gln Glu Val Gly Val Glu Glu Thr Pro Leu
            100                 105                 110

atg aac gtc gac tct att ctg gca gtt aaa aag tac ttc cag cgt atc 384
Met Asn Val Asp Ser Ile Leu Ala Val Lys Lys Tyr Phe Gln Arg Ile
        115                 120                 125

act ctg tac ctg acc gaa aag aaa tat tct ccg tgc gct tgg gaa gta 432
Thr Leu Tyr Leu Thr Glu Lys Lys Tyr Ser Pro Cys Ala Trp Glu Val
    130                 135                 140

gtt cgc gct gaa att atg cgt tct ttc tct ctg tct act aac ctg cag 480
Val Arg Ala Glu Ile Met Arg Ser Phe Ser Leu Ser Thr Asn Leu Gln
145                 150                 155                 160

gag cgt ctg cgc cgt aaa gaa taatag 507
Glu Arg Leu Arg Arg Lys Glu

                165

<210>  8
<211>  507
<212>  DNA
<213>  Artificial Sequence

<220>
<223>  Synthetic Construct

<400>  8
tacacactaa atggagtttg agtaagagaa ccattggcag cgcgagacta agacgaccgt      60

gtctacgcag cataaagggg caaatcgacg gactttctgg cagtgctgaa gccgaaaggc     120

gttcttctca agctaccgtt ggttaaggtc tttcgagtcc gttagagaca tgacgtgctt     180

tactaggttg tctggaagtt ggacaaaagg tgatttctgt cgagacgacg aaccctgctt     240

tcgaacgacc tcttcaagat gtgacttgac atagtcgtcg acttgctgga ccttcgtacg     300

cattaggtcc ttcaaccaca tcttctctga ggcgactact tgcagctgag ataagaccgt     360

caatttttca tgaaggtcgc atagtgagac atggactggc ttttctttat aagaggcacg     420

cgaacccttc atcaagcgcg actttaatac gcaagaaaga gagacagatg attggacgtc     480

ctcgcagacg cggcatttct tattatc                                         507

## Description

The application is a Continuation-In-part App'l of U. S. Serial No. 60/659,925, filed March 9, 2005; the contents of which are hereby incorporated in their entireties by reference into this application.

Throughout this application, various publications are referenced. Disclosures of these publications in their entireties are hereby incorporated by reference into this application in order to more fully describe the state of the art to which this invention pertains.

### FIELD OF THE INVENTION

This invention is related to a field of bioengineering. Specifically this invention relates to a recombinant super-compound interferon **(rSIFN-co)** or its equivalent with changed spatial configuration, high efficacy and low side effects. Therefore, high dose of rSIFN-co may be used. This invention also relates to a process to produce said super-compound interferon (rSIFN-co) or a pharmaceutical composition comprising said super-compound interferon (rSIFN-co) or its equivalent, and uses of said interferon or composition for anti-viral and anti-tumor therapy.

### BACKGROUND OF THE INVENTION

IFN-con is a new interferon molecule constructed with the most popular conservative amino acid found in natural human IFN-α subtypes using genetic engineering methods. United States Patent Nos. 4,695,623 and 4,897,471 have described it. IFN-con had been proven to have broad-spectrum IFN activity and virus- and tumor-inhibition and natural killer cell activity. United States Patent No. 5,372,808 by Amgen, Inc. addresses treatment Infergen^{®} (interferon alfacon-1). Chinese Patent No. 97193506. 8 by Amgen, Inc. addresses re-treatment of Infergen^{®} (interferon alfacon-1) on hepatitis C. Chinese Patent No. 98114663. 5 by Shenzhen Jiusheng Bio-engineering Ltd. addresses recombinant human consensus interferon-α treatment for hepatitis B and hepatitis C.

The United States Food and Drug Administration (FDA) authorized Amgen to produce Infergen^{®} (interferon alfacon-1) with E. Coll. for clinical hepatitis C treatment at the end of 1997.

Hepatitis B patients can be identified when detecting HBsAg and the HBeAg. IFN-α is commonly used in clinics to treat hepatitis B. IFN-α binds superficial cell membrane receptors, thus inhibiting DNA and RNA (ribonucleic acid) duplication and inducing some enzymes to prevent duplication of the virus in hepatitis-infected cells. All IFNs can inhibit DNA duplication of viruses, but they cannot inhibit the e and s antigen expression.

An outbreak of atypical pneumonia, referred to as severe acute respiratory syndrome (SARS) and first identified in Guangdong Province, China, has spread to several countries. Similar cases were detected in patients in Hong Kong, Vietnam, and Canada from February and March 2003. The World Health Organization (WHO) issued a global alert for the illness. In mid-March 2003, SARS was documented in health care workers and household members who had cared for patients with severe respiratory illness in the Far East. Many of these cases could be traced through multiple chains of transmission to one health care worker from Guangdong Province who visited Hong Kong, where he was hospitalized with pneumonia and died. By late April 2003, thousands of SARS cases and hundreds of SARS-related deaths from over 25 countries around the world were reported to WHO. Most of these cases occurred through exposure to SARS patients in household or health care settings. This invention provides a method to prevent and/or treat SARS.

This disclosure describes recombinant super-compound interferon **(rSIFN-co)**, method to produce the same and uses thereof. Particularly, the super-compound interferon disclosed herein is capable of inhibiting, preventing and/or treating the hepatitis viruses, SARS virus, or virus-induced upper respiratory diseases, the Influenza virus, for example Avian Influenza virus and Ebola virus.

In addition, **rSIFN-co** is effective in preventing and/or treating viral diseases and tumors with less side effects as compared to other available interferons.

### SUMMARY OF THE INVENTION

This invention provides a recombinant super-compound interferon **(rSIFN-co)** and its equivalent with changed spatial configuration, high efficacy and low side effects. Therefore, high dose of **rSIFN-co** may be used.

This invention provides an expression system comprising the vector comprising the gene which codes for the super-compound interferon or its equivalent. This invention also provides a host cell comprising the vector comprising the gene which codes for the recombinant super-compound interferon **(rSIFN-co)** or its equivalent. Said host cell may be eukaryotic or prokaryotic, such as E. Coli.

This invention provides a method for inhibiting, preventing or treating viral diseases, or for inhibiting or treating tumors in a subject comprising administering to the subject an effective amount of the super-compound interferon or its equivalent.

This invention provides the above-described method wherein super-compound interferon is administered orally, via vein injection, muscle injection, peritoneal injection, subcutaneous injection, nasal or mucosal administration, or by inhalation via a respirator.

This invention provides the method to prevent or treat viral diseases wherein the viral diseases is hepatitis A, hepatitis B, hepatitis C, other types of hepatitis, infections of viruses caused by Epstein-Barr virus, Human Immunodeficiency Virus (HIV), Ebola virus, Severe Acute Respiratory Syndrome Virus (SARS), Influenza virus, Cytomegalovirus, herpes simplex viruses, or other types of herpes viruses, papovaviruses, poxviruses, picornaviruses, adenoviruses, rhinoviruses, human T-cell leukemia viruses I, or human T-cell leukemia viruses II, or human T-cell leukemia virus III.

This invention provides the method to prevent or treat viral diseases wherein the viral diseases are Human Immunodeficiency Virus (HIV) and Ebola virus.

This invention provides a method for anti-hepatitis activities. It can inhibit HBV-DNA replication, HBsAg and HBeAg production.

This invention provides a method to prevent or treat upper respiratory infection diseases.

This invention provides a method to prevent or treat tumors or cancers wherein the tumor is skin cancer, basal cell carcinoma and malignant melanoma, renal cell carcinoma, liver cancer, thyroid cancer, rhinopharyngeal cancer, solid carcinoma, prostate cancer, stomach/abdominal cancer, esophageal cancer, rectal cancer, pancreatic cancer, breast cancer, ovarian cancer, and superficial bladder cancer, hemangioma, epidermoid carcinoma, cervical cancer, non-small-cell lung cancer, small-cell lung cancer, glioma, leucocythemia, acute leucocythemia and chronic leucocythemia, chronica myelocytic leukemia, hairy cell leukemia, lymphadenoma, multiple myeloma, polycythemia vera, or Kaposi's sarcoma.

This invention provides a method for preventing or treating virus-induced diseases in a subject comprising administering to the subject an effective amount of recombinant super-compound interferon or a functional equivalent thereof.

The super-compound interferon **(rSIFN-co)** may be administered orally, via vein injection, muscle injection, peritoneal injection, subcutaneous injection, nasal or mucosal administration, or by inhalation via a respirator.

This invention provides a method for inhibiting the causative agent of virus-induced diseases, comprising contacting the causative agent with an effective amount of super-compound interferon or its equivalent.

This invention also provides a method for inhibiting virus-induced diseases, comprising contacting an effective amount of the super-compound interferon with said virus or cells. This contact could be direct or indirect.

This invention provides a composition comprising an effective amount of the super-compound interferon capable of inhibiting, preventing or treating virus-induced diseases, and a suitable carrier.

This invention provides a pharmaceutical composition comprising an effective amount of the recombinant super-compound interferon capable of inhibiting, preventing or treating virus-induced diseases in a subject, and a pharmaceutically acceptable carrier.

This invention provides a method for preventing or treating tumors in a subject comprising administering to the subject an effective amount of recombinant super-compound interferon or a functional equivalent thereof.

This invention provides a method for inhibiting tumors, comprising contacting the causative agent with an effective amount of super-compound interferon or its equivalent.

This invention also provides a method for inhibiting tumors, comprising contacting an effective amount of the super-compound interferon with said virus or cells. This contact could be direct or indirect.

This invention provides a composition comprising an effective amount of the super-compound interferon capable of inhibiting, preventing or treating tumors, and a suitable carrier.

This invention provides a pharmaceutical composition comprising an effective amount of the recombinant super-compound interferon capable of inhibiting, preventing or treating tumors in a subject, and a pharmaceutically acceptable carrier.

### DETAILED DESCRIPTION OF THE FIGURES

**Figure 1****. rSIFN-co** cDNA sequence designed according to E. Coli. codon usage and deduced **rSIFN-co** amino acid sequence

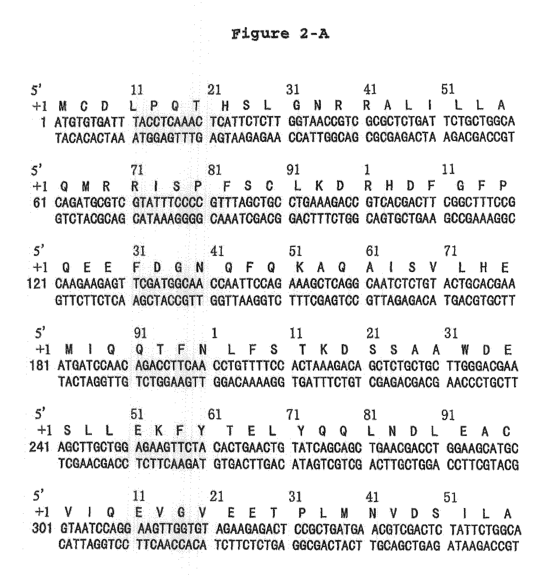

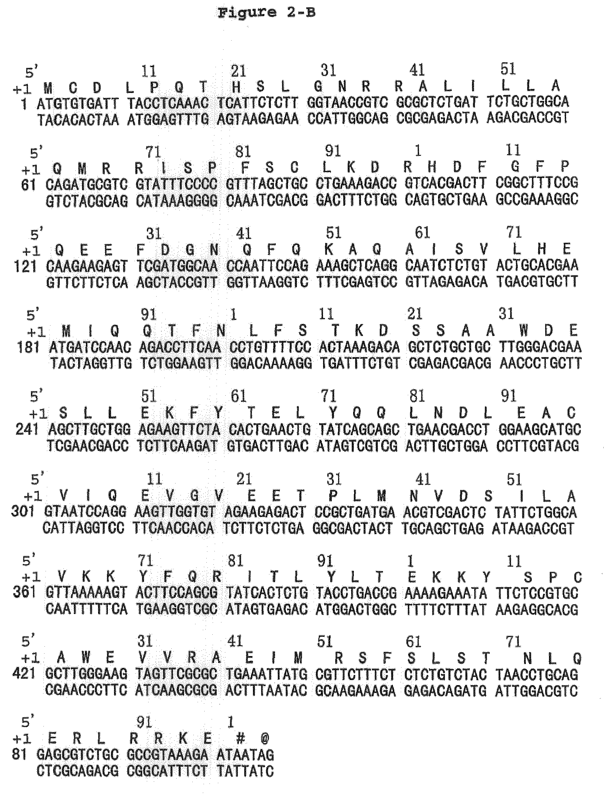

**Figures 2A-B****.** Sequences of another super-compound interferons

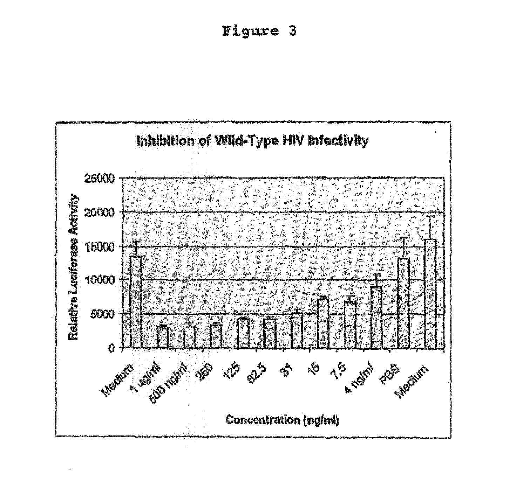

**Figure 3****.** Graph of Inhibition of Wild-Type HIV by **rSIFN-co** using EXCEL and Luciferase as Y axis and concentration of **rSIFN-co** as X axis. A clear inverse dose-dependent response has been shown.

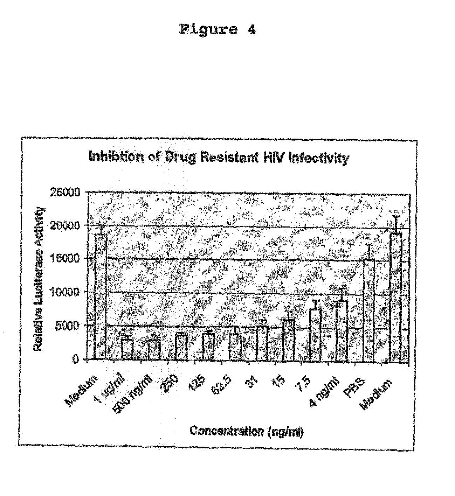

**Figure 4****.** Graph of Inhibition of Drug Resistant HIV by **rSIFN-co** using EXCEL and Luciferase as Y axis and concentration of **rSIFN-co** as X axis. A clear inverse dose-dependent response has been shown.

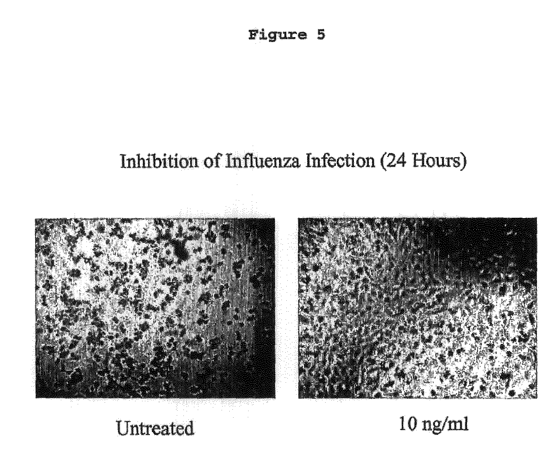

**Figure 5****. rSIFN-co** Inhibition of Influenza Virus: on the left, the control well is shown with Influenza virus added and without interferon, the cells had obvious CPE, such as rounding of cells, cell necroses, decrease in reflective light and sloughing off. On the right, the experimental wells is shown containing Influenza virus and **rSIFN-co** at concentration 10 nanogram per milliliter (ng/ml) had morphology comparable to normal cells.

### DETAILED DESCRIPTION OF THE INVENTION

### Recombinant Super-compound Interferon (rSIFN-co)

This invention provides a recombinant super-compound interferon **(rSIFN-co)** or an equivalent thereof with changed spatial configuration. This invention reveals that proteins with the same primary sequence might have different biological activities. As illustrated in this application, proteins with identical amino acid sequences may have different activities. The efficacy of these proteins may sometimes be improved and, sometimes, proteins with changed spatial configuration would reveal new function. Wei (2005; Publication No. WO 2005/02177 A2 ; Int'l App'l No. PCT/US2004/028068) provides a set of methods for modulating protein spatial configuration.

As defined herein, equivalents are molecules which are similar in function to the compound interferon. An equivalent could be a deletion, substitution, or replacement mutant of the original sequence. Alternatively, it is also the intention of this invention to cover mimics of the recombinant super-compound interferon **(rSIFN-co)**. Mimics could be a peptide, polypeptide or a small chemical entity.

The recombinant super-compound interferon **(rSIFN-co)** described herein includes but is not limited to interferon α, β, γ or ω. In an embodiment, it is IFN-1α, IFN-2β or other mutants.

In another embodiment, the recombinant super-compound interferon **(rSIFN-co)** disclosed has higher efficacy than α, β, γ, ω or a combination thereof and as compared to the interferons disclosed in United States Patent Nos. 4,695,623 and 4,897,471. This recombinant super-compound interferon (rSIPN-co) is believed to have unique secondary or tertiary structure, wherein the 3-dimensional change is the result of changes in its production process. Wei (2004; US 2004/0202641 A1; U. S. Serial No. 10/650,365) also describes this recombinant super-compound.

The recombinant super-compound interferon **(rSIFN-co)** described herein has spatial structure change(s) resulting from the changes of its production process.

### Lower Side Effects

The recombinant super-compound interferon **(rSIFN-co)** possesses lower side effects when compared with other interferons. These lower side effects allow for higher dosages to be used on patients in need of interferon treatments. These lower side effects open the possibility of using **rSIFN-co** for prevention and/or treatment of other diseases. Accordingly, this invention provides the recombinant super-compound interferon **(rSIFN-co)** with less side effects when administered to a subject.

This invention provides recombinant super-compound interferon **(rSIFN-co)** with less side effects as compared to all currently available interferons.

This invention further provides a method for treating or preventing viral diseases or tumors in a subject comprising administering to the subject an effective amount of the **rSIFN-co** with less side effects as compared to all currently available interferons. Therefore, high dose of **rSIFN-co** may be used. In an embodiment, the effective amount of recombinant super-compound interferon is in nanogram level.

### Process to Produce rSIFN-co

### Artificial Gene

This invention also provides artificial gene encoding for the super-compound interferon or its equivalent. It is within the ordinary skill to design an artificial gene. Many methods for generating nucleotide sequence and other molecular biology techniques have been described previously. See for example, Joseph Sambrook and David W. Russell, Molecular Cloning: A laboratory Manual, December 2000, published by Cold Spring Harbor Laboratory Press.

The recombinant super-compound interferon **(rSIFN-co)** may also be produced with its gene as artificially synthesized cDNA with adjustment of its sequence from the wild-type according to codon preference of E. Coll. Extensive discussion of said codon usage (preference) may be found in U. S. Patent No. 4, 695,623. See e. g. column 6, line 41 - column 7, line 35.

### Vector

This invention provides a vector comprising the gene which codes for the super-compound interferon or its equivalent. This invention provides an expression system comprising the vector comprising the gene which codes for the super-compound interferon or its equivalent. The cells include, but are not limited to, prokaryotic or eukaryotic cells. This invention also provides a host cell comprising the vector comprising the gene which codes for the recombinant super-compound interferon **(rSIFN-co)** or its equivalent.

This invention provides a method for producing a recombinant super-compound interferon **(rSIFN-co)** with changed spatial configuration and enhanced antiviral activity comprising steps of :
(a) Introducing nucleic acid molecule which codes for said interferon with preferred codons for expression to an appropriate host; and
(b) Placing the introduced host in conditions allowing expression of said interferon. This invention provides the method for producing recombinant super-compound interferon **(rSIFN-co)**, further comprising recovery of the expressed interferon.

### Expression System

The above-described recombinant super-compound interferon **(rSIFN-co)** may be produced by a high-efficiency expression system which uses a special promoter, enhancer or other regulatory element. In an embodiment the promoter is inducible. Said inducible promoter includes but is not limited to P_{BAD}, heat shock promoters or heavy metal inducible promoters. Heat shock promoters are activated by physical means, while other promoters are activated by chemical means, for example IPTG or Tetracyclin. IPTG is added to the cells to activate the downstream gene or removed to inactivate the gene. Tetracyclin is used to induce promoters or to regulate the strength of promoters. See http://www.bio.davidson.edu/courses/c)enomics/method/ plasmid inducible.html.

In an embodiment the promoter is P_{BAD}. Since early nineties, the properties of the mechanism of expression and repression of P_{BAD} by AraC have been studied extensively, and their interactions have been dissected at the molecular level. See Schleif, R. S. 1992 DNA looping. Annu. Rev. Biochem. 61:199-223. The AraC protein is both a positive and negative regulator, when present, it turns on the transcription from the P_{BAD} promoter, when absent, the transcription occurs at a very low rate. See Guzman, L. M. et al. (1995) J. Bact. 177: 4121-4130. The efficacy and mechanism of P_{BAD} promoter is well known by other ordinary skilled artisans and is commercially-available. See http://www.invitroqen.com/content/sfs/brochures/710 01619 pB AD bro.pdf

The commercially-available Invitrogen expression kit includes pBAD vectors' designed to provide precise control of expression levels. The araBAD promoter initiates gene expression. It's both positively and negatively regulated by the product of the araC gene, a transcriptional regulator that forms a complex with L-arabinose. In the absence of arabinose, the AraC dimer contacts the 02 and 11 half sites of the araBAD operon, forming a 210 bp DNA loop. For maximum transcriptional activation, two events are required: first, Arabinose binds to AraC. The protein releases the 02 site and binds the 12 site, which is adjacent to the 11 site. This releases the DNA loop and allows transcription to begin. Second, the cAMP activator protein (CAP)-cAMP complex binds to the DNA and stimulates binding of AraC to 11 and 12. Basal expression levels can be repressed by introducing glucose to the growth medium. Glucose acts by lowering cAMP levels, which in turn decreases the binding of CAP. As cAMP levels are lowered, transcriptional activation is decreased. Invitrogen' s pBAD vectors are specifically designed for maximum expression and ease of use.

Nine pBAD vectors are currently available: pBAD102/D-TOPO^{®}, pBAD202/D-TOPO^{®}, pBAD-TOPO^{®}, pBAD/Thio-TOPO^{®}, pBAD/His, pBAD/Myc-His, pBAD-DEST49, pBAD/gIII and pBAD/Thio-E. with the following features in all pBAD vectors:
1. araBAD promoter for dose-dependent regulation
2. araC gene for tight control of the araBAD promoter
3. Optimized ribosome binding site for increased translation efficiency
4. rrnB transcription termination region for efficient transcript

The inducible promoters include but are not limited to heat shock promoters or heavy metal inducible promoters.

This invention provides a process for production of recombinant super-compound interferon **(rSIFN-co)** comprising introducing an artificial gene with selected codon preference into an appropriate host, culturing said introduced host in an appropriate condition for the expression of said compound interferon and harvesting the expressed compound interferon.

The process may comprise extraction of super recombinant super-compound interferon **(rSIFN-co)** from fermentation broth, collection of inclusion bodies, denaturation and renaturation of the harvested protein.

The process may maintain the high efficacy even when the recombinant super-compound interferon **(rSIFN-co)** is used with an agent and in a particular concentration. The process also comprises separation and purification of the recombinant super-compound interferon **(rSIFN-co)**. The process further comprises lyophilization of the purified recombinant super-compound interferon (rSIFN-co). The process comprises production of liquid injection of recombinant super-compound interferon **(rSIFN-co)**.

In one embodiment, recombinant super-compound interferon **(rSIFN-co)** was produced with recombinant techniques. On the condition of fixed amino acid sequence, the IFN DNA was redesigned according to the E. Coli. codon usage and then the rSIFN-co gene was artificially synthesized. rSIFN-co cDNA was cloned into the high-expression vector of E. Coli. by DNA recombinant techniques, and a high expression of rSIFN-co was gained by using of induce/activate-mechanism of L-arabinose to activate the transcription of P_{BAD} promoter.

Compared with usual thermo-induction, pH induction and IPTG induction systems of genetic engineering, arabinose induction/activation system has some advantages: (1) Common systems relieve promoter function by creating a "derepression" pattern. Promoters then induce downstream gene expression. Temperature and pH change and the addition of IPTG cannot activate promoters directly. In the system disclosed herein, L-arabinose not only deactivates and represses but also activates the transcription of P_{BAD} promoter which induces a high expression of rSIFN-co. Therefore, the arabinose induction/activation system is a more effective expression system. (2) The relationship between Exogenous and L-arabinose dosage is linear. This means the concentration of arabinose can be changed to adjust the expression level of the exogenous gene. Therefore, it is easier to control the exogenous gene expression level in E. Coli. by arabinose than by changing temperature and pH value. This characteristic is significant for the formation of inclusion bodies. (3) L-arabinose is resourceful, cheap and safe, which, on the contrary, are the disadvantages of other inducers such as IPTG.

This embodiment creates an effective and resistant rSIFN-co-expressing E. Coli. engineering strain with an L-arabinose induction/activation system. The strain is cultivated and fermented under suitable conditions to harvest the bacterial bodies. Inclusion bodies are then purified after destroying bacteria and washing repeatedly. The end result, mass of high-purity, spatial-configuration-changed rSIFN-co protein for this invention and for clinical treatment, was gained from denaturation and renaturation of inclusion bodies and a series of purification steps. Said purification would not effect the biological activity of the purified protein.

The above-described recombinant super-compound interferon **(rSIFN-co)** possesses anti-viral or anti-tumor activity, and; therefore, is useful in inhibiting, preventing and treating viral diseases, inhibiting or treating tumors, or cancers.

### Viral Diseases

This invention provides a method for treating or preventing viral diseases or tumors in a subject comprising administering to the subject an effective amount of the recombinant super-compound interferon **(rSIFN-co)** or its equivalent. Wei (2005; Int'l Publication No. WO 2005/034853 A2) provides a method for preventing or treating Severe Acute Respiratory Syndrome in a subject.

As used herein, viral diseases include, but are not limited to, hepatitis A, hepatitis B, hepatitis C, other types of hepatitis, infections caused by Epstein-Barr virus, Human Immunodeficiency Virus (HIV), Ebola virus, Severe Acute Respiratory Syndrome Virus (SARS), Influenza virus, Cytomegalovirus, herpes simplex viruses, other herpes viruses, papovaviruses, poxviruses, picornaviruses, adenoviruses, rhinoviruses, human T-cell leukemia virus I, human T-cell leukemia virus II, or human T-cell leukemia virus III.

In an embodiment, the effective amount is at nanogram level. In another embodiment, the virus is Human Immunodeficiency Virus and the effective amount is as low as 4 nanograms per milliliter. In another embodiment, the virus is Influenza and the effective amount is as low as 10 nanogram per milliliter.

### Inhibition of DNA Replication and Secretion of of HBsAg and HBeAg of Hepatitis B Virus.

The recombinant super-compound interferon **(rSIFN-co)** inhibits the DNA duplication and secretion of HBsAg and HBeAg of Hepatitis B Virus.

### Severe Acute Respiratory Syndrome Virus (SARS)

This invention provides a method for preventing or treating Severe Acute Respiratory Syndrome, or virus-induced upper respiratory diseases, of a subject comprising administering to the subject an effective amount of recombinant super-compound interferon **(rSIFN-co)** or a functional equivalent thereof. In an embodiment of the above method, the interferon is α, β, γ, ω or a combination thereof.

The recombinant super-compound interferon **(rSIFN-co)** may be administered orally, via vein injection, muscle injection, peritoneal injection, subcutaneous injection, nasal or mucosal administration, or by inhalation via a spray or a respirator. In an embodiment **rSIFN-co** is administered subcutaneously or intramuscularly at a dose of higher than or equal to 10 Million International Unit per square meter of surface area. In another embodiment **rSIFN-co** is administered subcutaneously or intramuscularly at a dose of higher than or equal to 20 Million International Unit per square meter of surface area. In an embodiment, the interferon is delivered by a spray device. In a specific embodiment, the device is described in Figure 11. In one of the embodiments, the interferon is lyophilized.

This invention provides a method for inhibiting the causative agent of Severe Acute Respiratory Syndrome, or virus-induced upper respiratory diseases, comprising contacting the agent with an effective amount of recombinant super-compound interferon **(rSIFN-co)** or its equivalent.

It is determined that the causative agent of SARS is a virus. See eg. Rota et al (2003), Characterization of a Novel Coronavirus Associated with Severe Acute Respiratory Syndrome. Science 1085952 www. sciencexpress. org and Marra, et al. (2003), The Genome Sequence of the SARS-Associated Coronavirus. Science 1085853 www. sciencexpress. org.

This invention also provides a method for inhibiting Severe Acute Respiratory Syndrome virus or Severe Acute Respiratory Syndrome virus-infected cells, or virus-induced upper respiratory diseases, or cells infected with viruses capable of inducing upper respiratory diseases, comprising contacting an effective amount of the recombinant super-compound interferon **(rSIFN-co)** with said virus or cell. This contact could be direct or indirect.

This invention provides a composition comprising an effective amount of the recombinant super-compound interferon **(rSIFN-co)** capable of inhibiting Severe Acute Respiratory Syndrome virus or Severe Acute Respiratory Syndrome virus-infected cells, or virus-induced upper respiratory diseases, or cells infected with viruses capable of inducing upper respiratory diseases, and a suitable carrier.

This invention provides a composition comprising an effective amount of the super-compound interferon capable of preventing or treating Severe Acute Respiratory Syndrome, or virus-induced upper respiratory diseases, of a subject and a suitable carrier.

This invention provides a pharmaceutical composition comprising an effective amount of the recombinant super-compound interferon **(rSIFN-co)** capable of inhibiting Severe Acute Respiratory Syndrome virus or Severe Acute Respiratory Syndrome virus-infected cells, or virus-induced upper respiratory diseases, and a pharmaceutically acceptable carrier.

This invention provides a pharmaceutical composition comprising an effective amount of the recombinant super-compound interferon (rSIFN-co) capable of preventing or treating Severe Acute Respiratory Syndrome, or virus-induced upper respiratory diseases, in a subject and a pharmaceutically acceptable carrier.

This invention provides a device to deliver the above-described pharmaceutical composition.

In a preferred embodiment, the subject is a human. As it can easily be appreciated, the super-compound interferon can be used in other animals or mammals.

This invention provides a method for preventing Severe Acute Respiratory Syndrome or virus-induced upper respiratory diseases, in humans comprising application of the super-compound interferon three times a day via a spray which contains twenty micrograms of interferon, equal to ten million units of activity in three milliliter.

### Viral Upper Respiratory Infection (VURI)

Viral upper respiratory infection, alternative names common cold, colds. This is a contagious viral infection of the upper respiratory tract characterized by inflammation of the mucous membranes, sneezing, and a sore throat. It is usually caused by over 200 different viruses, known as rhinoviruses. Colds are not caused by the same viruses responsible for Influenza. Colds are spread through droplets from the coughing or sneezing of others with a cold or by hand contact with objects contaminated by someone with a cold. The incidence of colds is highest among children, and the incidence decreases with age because immunity to the virus causing the cold occurs after the illness. Gradually, immunity to a wide variety of viruses that cause colds is developed in adults. Children may have 10 colds a year, and adults may have 3 colds a year.

If a patient presents with a viral URI, the spectrum of remedies is extensive. Since most of these infections are self-limiting, clinicians usually recommend rest and fluids, but other treatments include environmental and nutritional therapies, over-the-counter and prescription decongestant and antihistamine products, new antihistamine and anticholinergic nasal formulations, and antibiotics. See

### http://www.physsportsmed.com/issues/1998/02feb/swain. htm (2005)

### Prevention and Treatment of Upper Respiratory Tract Infections (URI)

Nearly 70-80% URI are caused by viruses such as respiratory Syncytical virus, adenovirus, rhinovirous, cox-sackie virus, corona virus and its variant, influenza A virus and its variant, influenza B virus and its variant, parainfluenza virus and its variant, or enterovirus and its variant. A main cause of URI in adults is from rhinovirous. For children, respiratory syncytical virus and parainfluenza virus are two leading causes of URI.

Recombinant super-compound interferon (rSIFN-co) plays an important role in the fight against virus that causes URI. Super-compound interferon gains its anti-virus affects mainly via two mechanisms:
1. Attach to surface of sensitive cells and induce them to produce anti-virus protein, then block the duplication and reproduction of viruses in *vivo.*
2. recombinant super-compound interferon (rSIFN-co) can adjust immune response, including T-cell immune response, activity of NK cell, the phagocytosis function of monokaryon, and even formation of some antibodies in *vivo.*

In treatment for URI, recombinant super-compound interferon (rSIFN-co) can be directly applied to the affected area via a spray or a respiration. This method of treatment allows the interferon to reach the target cells first hand.Consequently, marketing the supply as a spray, rather than via oral or injection, would be safer and more effective for administrating the interferon.

### Prevention and Treatment of SARS

With the consent of the *Sichuan* (a province in China) *working group on SARS prevention and control,* the distribution of recombinant super-compound interferon (rSIFN-co) began in May of 2003. Super-compound interferon spray was allocated to doctors and nurses in hospitals, populated areas with a high risk for SARS, and to the *National research group on prevention and control* of *SARS.* Among the 3,000 users as of December 19, 2003, there were no reports of any side effects connected to the use of the spray. Furthermore, none of the doctors and nurses, the people of Sichuan Province, or other organizations that have used the Super-compound interferon spray has been infected by SARS.

Therefore, this invention provides a method for inhibiting, preventing or treating virus replication or virus-infected cells by contacting said virus or infected cells with an effective amount of the recombinant super-compound interferon (rSIFN-co) or its equivalent.

### Prevention and Treatment of Tumors

This recombinant super-compound interferon (rSIFN-co) is useful in inhibiting, preventing or treating the following cancers or tumors:

| | | |
|---|---|---|
| Cancer | Skin Cancer | Basal Cell Carcinoma |
| | | Malignant Melanoma |
| | Renal cell carcinoma | |
| | Liver Cancer | |
| | Thyroid Cancer | |
| | Rhinopharyngeal Cancer | |
| | Solid Carcinoma | Prostate Cancer |
| | | Stomach/Abdominal Cancer |
| | | Esophageal Cancer |
| | | Rectal Cancer |
| | | Pancreatic Cancer |
| | | Breast Cancer |
| | Ovarian Cancer & Superficial Bladder Cancer | |
| | Hemangioma | |
| | Epidermoid Carcinoma | Cervical Cancer |
| | | Non-small Cell Lung Cancer |
| | | small Cell Lung Cancer |
| | | Glioma |
| Malignant | Leucocythemia | Acute Leucocythemia |
| Hemal Disease | | Chronic Leucocythemia |
| | Chronic Myelocytic Leukemia | |
| | Hairy Cell Leukemia | |
| | Lymphadenoma | |
| | Multiple Myeloma | |
| | Polycythemia Vera | |
| Others | Kaposi's Sarcoma | |

Accordingly, this invention provides a method for inhibiting tumor or cancer cell growth by contacting the recombinant super-compound interferon (rSIFN-co) or its equivalent with said tumor or cancer cells.

### Formulation and Route of Administration

This invention also provides the produced super-compound interferon by the above processes.

This invention provides a composition comprising recombinant super-compound interferon (rSIFN-co) or its equivalent and a suitable carrier.

This invention provides a pharmaceutical composition comprising the recombinant super-compound interferon (rSIFN-co) or its equivalent and a pharmaceutically acceptable carrier.

This invention provides the above-described method wherein recombinant super-compound interferon (rSIFN-co) was administered via orally via vein injection, muscle injection, peritoneal injection, subcutaneous injection, nasal or mucosal administration, or by inhalation via a spray or a respirator.

This invention provides the above-described method wherein recombinant super-compound interferon (rSIFN-co) was administered following the protocol of injections of 9µg,15µg or 24 µg every two days,3 times a week,for 24 weeks.

It was surprising to find that recombinant super-compound interferon (rSIFN-co),the spatial structure of which has been changed, is not only a preparation to inhibit the DNA duplication of hepatitis B, but to inhibit the secretion of HBsAg and HBeAg on 2.2.15 cells.

One objective of this invention is to offer a preparation of recombinant super-compound interferon (rSIFN-co) to directly inhibit the DNA duplication of hepatitis B viruses and the secretion of HBeAg and HBsAg of hepatitis B and decrease them to normal levels.

### Formulation

The following are some rSIFN-co preparations: tablets, capsules, liquids for oral consumption, pastes, injections, sprays, suppositories, and solutions. Injections are recommended. It is common to subcutaneously inject or vein-inject the medicine. The medicine carrier could be any acceptable medicine carrier, including carbohydrates, cellulosum, adhesive, collapse, emollient, filling, add-dissolving agent, amortization, preservative, thickening agent, matching, etc.

This invention also provides a pharmaceutical composition comprising the above composition and a pharmaceutically acceptable carrier.

For the purposes of this invention, "pharmaceutically acceptable carriers" means any of the standard pharmaceutical carriers. Examples of suitable carriers are well known in the art and may include, but are not limited to, any of the standard pharmaceutical carriers, such as a phosphate buffered saline solution and various wetting agents. Other carriers may include additives used in tablets, granules, capsules, etc. Typically such carriers contain excipients such as starch, milk, sugar, certain types of clay, gelatin, stearic acid or salts thereof, magnesium or calcium stearate, talc, vegetable fats or oils, gum, glycols or other known excipients. Such carriers may also include flavor and color additives or other ingredients. Compositions comprising such carriers are formulated by well-known conventional methods.

### Increase of the Half-life of rSIFN-co

### Pegylation

Pegylation is the process by which polyethylene glycol chains are attached to protein and peptide drugs to increase pharmacokinetics by shielding these proteins and peptide drugs from proteolytic enzymes. See Harris and Chess, Effect of pegylation on pharmaceuticals. Nat Rev Drug Discov. 2003 Mar;2(3):214-21.

Pegylations is a well-established method for increasing the circulating half-life of protein and liposomal pharmaceuticals based on large hydrodynamic volume of polyethylene glycols. These polyethylene glycols shield the proteins and peptide drugs from renal clearance, enzymatic degradation and immune system recognition, thus their half-life and making them more acceptable to patients. See Molineux, *Pegylation: engineering improved pharmaceuticals for enhanced therapy.* Cancer Treat Rev. 2002 Apr;28 Suppl A:13-6.The author concludes that pegylation has beneficial effect on the quality of life of cancer patients.

Pegylation of the interferon increases the amount of time the interferon remains in the body by increasing the size of the interferon molecule by decreasing the rate of absorption, prolonging the half-life and the rate of interferon clearance. Thus, the duration of biological activity is increased with pegylated interferon over nonpegylated interferon, thus providing an advantage over nonpegylated interferons with less frequent administration and comparable tolerability. The author states that monotherapy with pegylated interferon produces a better response in some patients than monotherapy with the nonpegylated formulation. See Baker, Pegylated Interferons. Rev Gastroenterol Disord. 2001;1(2):87-99.

### Sustained Release or Controlled Release

Sustained release delivery matrices and liposomes maybe used with rSIFN-co to create sustained release and controlled release formulation. See Robinson and Talmadge, Sustained Release of Growth Factors. In Vivo 2002 Nov-Dec;16(6):535-40.The authors state that both pegylation and sustained release delivery matrices and liposomes improve the pharmacokinetic and pharmacodynamic properties of recombinant molecules, and thus by improving clinical efficacy these approaches increase patient compliance.

This invention provides recombinant super-compound interferon (rSIFN-co) comprising an agent or encapsulated by an agent, capable of affecting the half-life or delivery of said interferon. In an embodiment this agent is polyethylene glycol (PEG).

This invention further provides a method for treating or preventing viral diseases or tumors in a subject comprising administering to the subject an effective amount of the recombinant super-compound interferon (rSIFN-co) or its equivalent comprising an agent or encapsulated by an agent, capable of affecting the half-life or delivery of said interferon. In an embodiment this agent is polyethylene glycol (PEG).

This invention will be better understood from the examples which follow. However, one skilled in the art will readily appreciate that the specific methods and results discussed are merely illustrative of the invention as described more fully in the claims which follow thereafter.

### EXPERIMENTAL DETAILS

IFN-con is a new interferon molecule constructed according to conservative amino acids in human IFN-α subtype using genetic engineering methods. It has been proven that IFN-con has broad-spectrum IFN activity, such as high antivirus and tumor inhibition activity, especially for effectively treating hepatitis C.

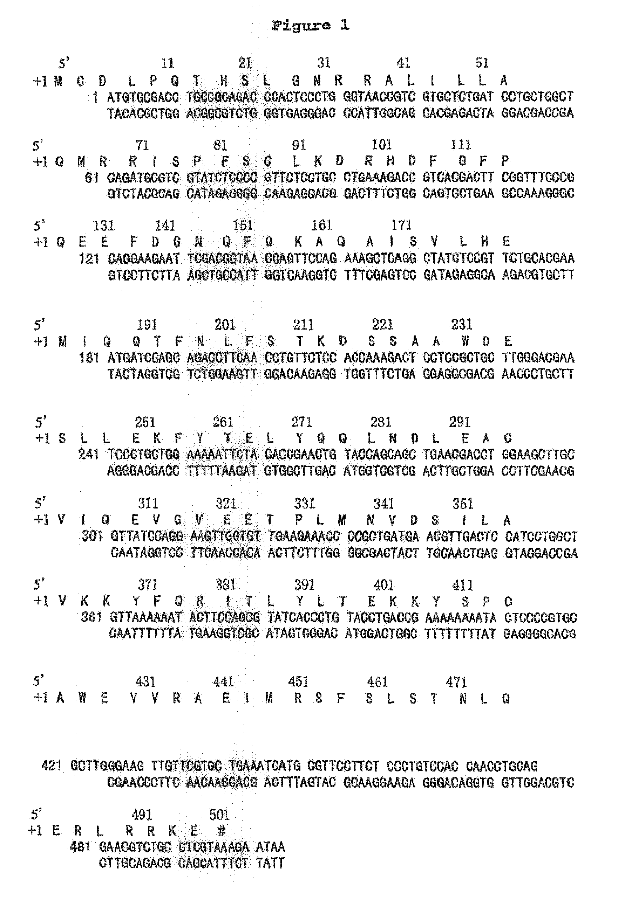

E. Coli. codon was used to redesign rSIFN-co cDNA and then artificially synthesize cDNA of rSIFN-co from published Infergen^{®} (interferon alfacon-1) DNA sequences and deduced amino acid sequences (Figure 1).

In order to get pure rSIFN-co protein, rSIFN-co cDNA was cloned into E. Coli. high-expression vector, and L-arabinose, which can activate strong P_{BAD} promoter in vectors, was used to induce high expression of rSIFN-co gene.

### EXAMPLE 1

### Effects of Recombinant Super-compound interferon (rSIFN-co) on Ebola virus.

Background: Ebola virus is a notoriously deadly virus that causes fearsome symptoms, the most prominent being high fever and massive internal bleeding. Ebola virus kills as many as 90% of the people it infects. It is one of the viruses capable of causing hemorrhagic (bloody) fever. There is no specific treatment for the disease. Currently, patients receive supportive therapy. This consists of balancing the patient's fluids and electrolytes, maintaining their oxygen level and blood pressure, and treating them for any complicating infections. Death can occur within 10 days of the onset of symptoms.

### 1. Materials

1.1 Drugs: rSIFN-co, provided by Sichuan Biotechnology Research Center.
1.2 Virus: Ebola, supplied by The Academy of Military Medical Science, Institute of Microbiology Epidemiology.
1.3 Safety level of experiment: Viral experiments were carried under Biological Laboratory Safety System level 3.
1.4 Animals: 60 BALB/c mice

### 2 Method

2.1 60 mice were randomly separated into 6 groups, each group consisting of 10 mice. Group 1 was treated with 1 µg/ of rSIFN-co on the day of inoculation with Ebola virus. Group 2 was treated with 1 µg/ of rSIFN-co day one (1) after inoculation with Ebola virus. Group 3 was treated with 1 µg/ of rSIFN-co on the day two (2) after inoculation with Ebola virus. Group 4 was treated with 1 µg/ of rSIFN-co on day three (3) after inoculation with Ebola virus. Group 5 was treated with 1 µg/ of rSIFN-co on day four (4) after inoculation with Ebola virus. Group 6 was not treated with rSIFN-co and this is designated as the control group.
2.2 Administration of the medication: 1 µg/ of rSIFN- co was administered once a day for six (6) consecutive days.

### 3 Results

All ten (10) mice in group 6 (control group) died. All mice in groups one (1) , two (2) and three (3) survived with no observable toxic effect. In groups four (4) and five (5), showed some effects.

### 4 Conclusion

Clearly these result show effectiveness of rSIFN-co against Ebola virus.

### EXAMPLE 2

### Anti-HIV Effects of Recombinant Super-compound interferon (rSIFN-co).

### 1. Materials

1.1 Wild-Type HIV
1.2 Drug Resistant HIV
1.3 293-CD4-CCR5 cells
1.4 DMEM, Gibco
1.5 Fetal Bovine Seru, Gibco
1.6 rSIFN-co provided by Sichuan Biotechnology Research Center
1.7 96-well plate, NUNC
1.8 CO₂ incubator
1.9 Laminar Flow Hood
I.IO Fluorometer
1.11 UV Absorbance Meter
1.12 Others

### 2. Method

2.1 293-CD4-CCR5 cells in exponential (log) phase were obtained, digested with 0.25% pancreatin, stained with Trypan blue stain to determine cell number and diluted with DMEM to concentration of 2 OxIO⁵ cells per milliliter (cell/ml) .
2.2 Each well of 96-well plate was filled with 100 µl (microliters) of 293-CD4-CCR5- DMEM suspension solution. The plate was placed into 5% carbon dioxide incubator at 37 degrees Celsius and observed the next day seventy percent (70%) of basal area of the well were recovered.
2.3 After supernatant was removed, IOOµl (microliters) of different concentrations of rSIFN- co were added to each well. Two controls were used: Phosphate Buffered Saline (PBS) and Growth Media.
2.4 The plate was placed into carbon dioxide incubator at 37 degrees Celsius for approximately18 to 20 hours.
2.5 Experimental wells: Different concentrations of the Wild-Type HIV and Drug Resistant HIV viruses were placed into each well at 100 µl (microliters) per well.
   Control wells: No virus was added, only IOOµl (microliters) of DMEM per well.
2.6 The plate was placed into carbon dioxide incubator at 37 degrees Celsius for approximately 24 hours.
2.7 Routine Luciferase assay was performed and protein concentrations of the supernatants were measured. Luciferase was measured in RLU/mg units.

### 3 Results

rSIFN-co can inhibit HIV at level of ≥ 4 nanograms per milliliter (ng/ml) . See Table 4 and Figures 14-15. When using Luciferase as Y axis and concentration of rSIFN-co as X axis, using EXCEL, it is clear that at level of rSIFN-co ≥ 4 nanograms per milliliter (ng/ml), the level of Luciferase activities are obviously lower than in PBS and Medium controls. A clear inverse dose-dependent response has been shown.

**Table 4**

| **Comparison of Inhibition of Wild-Type HIV and Drug Resistant HIV by rSIFN-co** | | |
|---|---|---|
| Concentration of rSIFN-co | Luciferase Assay | |
| | Wild-Type HIV | Drug Resistant HIV |
| Medium | 13500+2000 | 18000+2000 |
| 1 µg/ml | 3000+200 | 2800+800 |
| 500 ng/ml | 3000+600 | 2800+900 |
| 250 ng/ml | 3400+400 | 4000+600 |
| 125 ng/ml | 4300+200 | 4100+600 |
| 62.5 ng/ml | 4300+400 | 4100+1000 |
| 31 ng/ml | 5000+800 | 5100+800 |
| 15 ng/ml | 7200+400 | 6000+1500 |
| 7.5 ng/ml | 7000+800 | 7700+1300 |
| 4 ng/ml | 9000+2000 | 8900+2000 |
| PBS | 13000+3000 | 15100+2300 |
| Medium | 16000+3600 | 19000+2500 |

### 4 Conclusion: rSIFN-co is effective against both: Wild-Type HIV and Drug Resistant HIV.

### EXAMPLE 3

**Anti-Influenza Effects of Recombinant Super-compound interferon (rSIFN-co).**

### 1. Materials

1.1. 10-day old chick embryonic membrane cells
1.2. SIFN-co provided by Sichuan Biotechnology Research Center
1.3. Influenza virus provided by Molecular Biology Department of Institute of microbiology and epidemiology, Academy of Military Medical Science.
1.4. DMEM, Gibco
1.5. Newborn Calf Serum
1.6. 96-well plate, NUNC
1.7. CO₂ incubator
1.8. Laminar Flow Hood
1.9. Inverted Microscope
1.10. Others

### 2. Method

2.1 10 -day old chick embryonic membrane cell in exponential (log) phase were obtained, digested with 0.25 % pancreatin, stained with Trypan blue stain to determine cell number and diluted with DMEM to concentration of 2.Ox10⁵ cells per milliliter (cell/ml).
2.2 Each well of 96-well plate was filled with 100 µl (microliters) of 293-CD4-CCR5- DMEM suspension solution. The plate was placed into carbon dioxide incubator at 37 degrees Celsius. The next day cells grew to a monolayer.
2.3 After supernatant was removed, 100 µl (microliters) of different concentrations of rSIFN-co were added to each well. Two control wells: No rSIFN-co was added
2.4 The plate was placed into carbon dioxide incubator at 37 degrees Celsius for approximately 18 to 20 hours.
2.5 Experimental wells: Different concentrations of the Influenza virus were placed into each well at100 microliters (µl) per well.
   Control wells: No Influenza virus was added, only IOOµl (microliters) of DMEM per well.
2.6 The plate was placed into carbon dioxide incubator at 37 degrees Celsius for approximately 24 hours.
2.7 Cells were observed under inverted microscope.

### 3. Results

3.1 Under inverted microscope, the cells in the control well with Influenza virus added and without interferon had obvious CPE, such as rounding of cells, cell necroses, decrease in reflective light and sloughing off.
3.2 Cells from the experimental wells containing rSIFN-co at concentration ≥ 10 nanogram per milliliter (ng/ml) had no CPE and morphology comparable to normal cells. See Figure 16.
3.3 Control Wells without Influenza virus added and without interferon did not have any CPE.

### 4 Conclusion

At concentration ≥ 10 nanogram per milliliter (ng/ml) rSIFN-co is effective against Influenza virus.

### EXAMPLE 4

### Clinical Observation of a Lung Cancer Subject

A subject was admitted on October 7, 2005 for non small cell lung cancer. After a month of Chemotherapy treatment, no improvement was observed in the imaging examinations on October 22, 2005 and October 25, 2005. No progress was seen in the examination on January 4, 2006 as well. The subject was treated with super-compound interferons for one month. The regimen is as follows: The subject was given injections every other day. The first time, the subject was given a dosage of 9 micrograms. In all subsequent injections, the dosage was doubled to 18 micrograms. An examination on February 23, 2006 showed rapid improvement.

## Claims

1. A recombinant interferon for use as a medicament for preventing and/or treating a viral disease caused by Ebola virus, wherein the interferon is encoded by the polynucleotide sequence shown in Figure 1.

2. The recombinant interferon of claim 1, which inhibits the DNA duplication and secretion of HBsAg and HBeAg of Hepatitis B Virus.

3. The recombinant interferon of claim 2, which inhibits the DNA duplication and secretion of HBsAg and HBeAg of Hepatitis B Virus in an in vitro way.

4. The recombinant interferon of any one of claims 1-3, further comprising an agent or encapsulated by an agent, capable of affecting the half-life or delivery of said interferon.

5. The recombinant interferon of claim 4, wherein the agent is polyethylene glycol (PEG).

6. The recombinant interferon of any one of claims 1-5, wherein the interferon is administered orally, via vein injection, muscle injection, peritoneal injection, subcutaneous injection, nasal or mucosal administration, or by inhalation via a respirator.

7. A pharmaceutical composition for use for preventing and/or treating a viral disease caused by Ebola virus comprising an effective amount of the recombinant interferon of any one of claims 1-6 and a pharmaceutically acceptable carrier.
